# EUROPEAN PATENT SPECIFICATION

(11) **EP 3 910 313 B1**
(45) Date of publication and mention of the grant of the patent: **30.04.2025**
(21) Application number: 19909471.5
(22) Date of filing: 31.12.2019
(51) Int. Cl.: G01N 1/40, B01D 53/04, H05B 3/20, H05B 3/40

(54) **SAMPLE CONCENTRATOR TUBE HAVING HEAT-RESISTANT PLANAR HEATING ELEMENT ADHERED THERETO, ANALYSIS DEVICE COMPRISING SAME, AND ANALYSIS METHOD USING SAME**
PROBENKONZENTRATORROHR MIT DARAN ANHAFTENDEM HITZEBESTÄNDIGEM EBENEM HEIZELEMENT, ANALYSEVORRICHTUNG DAMIT UND ANALYSEVERFAHREN MIT VERWENDUNG DAVON
TUBE CONCENTRATEUR D'ÉCHANTILLON AYANT UN ÉLÉMENT CHAUFFANT PLAN RÉSISTANT À LA CHALEUR COLLÉ SUR CELUI-CI, DISPOSITIF D'ANALYSE LE COMPRENANT, ET PROCÉDÉ D'ANALYSE L'UTILISANT

(30) Priority: 09.01.2019 KR 20190002906
(43) Date of publication of application: 17.11.2021
(73) Proprietor: Bioneer Corporation, Daejeon 34302 (KR)
(72) Inventor: PARK, Han Oh, Sejong-si 30151 (KR); KIM, Taeman, Mungyeong-si Gyeongsangbuk-do 36977 (KR); KIM, Jae Ha, Daejeon 34169 (KR)
(74) Representative: Kraus & Lederer PartGmbB
(86) International application number: PCT/KR2019/018823
(87) International publication number: WO 2020/145563

(56) References cited:
- CN-A- 101 868 072
- KR-A- 20050 059 364
- KR-A- 20110 070 509
- KR-A- 20120 057 056
- KR-A- 20170 104 753
- US-A1- 2009 096 348
- US-A1- 2012 216 597
- US-A1- 2012 216 597
- US-A1- 2013 061 756

## Description

### [Technical Field]

The present invention relates to a sample concentrator tube having a heat-resistant planar heating element adhered thereto, an analysis device comprising the same, and an analysis method using the same.

### [Background Art]

As a method of measuring a gas sample in the air, generally, an analysis device such as gas chromatography (GC), a mass spectrometer (MS), or the like, has been used, but it is difficult to measure a trace amount of gas due to a limit of detection of a detector.

Therefore, various studies for improving sensitivity of a detector sensor itself have been conducted. However, since sensitivity above a certain level is easily affected by disturbance, noise is inevitably increased. In order to solve above-mentioned problem, a sample concentration device for concentrating a low-concentration sample to a high concentration is used in a high-sensitivity analysis device such as a gas chromatography, an ion mobility spectrometry, or the like.

Specifically, in the sample concentration device, a cold trap method and a method using a sorbent are used. The cold trap method is a method of cooling a concentrator tube to a low temperature to concentrate and collect a gas sample and then raising a temperature to perform vaporization in a gas form, and the method of using a sorbent is a method of adsorbing a volatile material to a sorbent at room temperature or lower and desorbing the volatile material therefrom by heating to a high temperature.

In a sample concentration device using a sorbent, in order to prevent thermal damage of the sorbent for adsorbing a sample, it is ideal to rapidly, uniformly, and accurately raise a temperature to a desorption temperature. In a case where an amount of a sample to be detected is significantly small, it is determined whether or not to detect the sample by adsorption/desorption characteristics, and the high performance as described above is thus required. Particularly, a short thermal desorption time and a high heating rate are required for efficient analysis, but there is a need to prevent the sorbent from being degenerated by thermal shock applied to the sorbent due to over-heating. For example, in a case where significantly high heat is applied to the sorbent, the sorbent or the adsorbed material is degenerated, such that it is impossible to suitably analyze the adsorbed material.

Sample concentration devices in the related art have been developed with a structure in which a heating wire was wound on an outer surface of a tube accommodating a sample, and at the time of heating for desorption of a sample, the heating wire was linearly expanded, such that close adhesion with the tube was decreased. Therefore, it was difficult to precisely control an actual sample temperature, and there was a limit to efficiently transferring heat to a concentrator tube.

### [Related Art Document]

### [Patent Document]

### KR10-1814964B1 (December 27, 2017)

Among further prior art, US 2013/0061756 A1 discloses an adsorption unit, including an electrical heating substrate defined with a fluid channel therein, and an adsorptive material layer formed on the electrical heating substrate to contact the fluid channel for adsorbing moisture or volatile organic compounds in a gas flow through the fluid channel.

### [Disclosure]

### [Technical Problem]

An object of the present invention is to provide a sample concentrator tube having a heat-resistant planar heating element adhered thereto and capable of rapidly and precisely raising a temperature to a desorption temperature of a sample sorbent, an analysis device comprising the same, and an analysis method using the same.

Another object of the present invention is to provide a sample concentrator tube having a heat-resistant planar heating element adhered thereto and is capable of applying a uniform temperature to a sample sorbent by significantly deceasing a temperature difference between a large number of sorbents provided in the tube so that thermal energy is effectively transferred from an outer surface of the tube even though the sorbent is located in any position from an inner surface of the tube to a central portion of the tube, an analysis device comprising the same, and an analysis method using the same.

Still another object of the present invention is to provide a sample concentrator tube having a heat-resistant planar heating element adhered thereto and capable of minimizing chemical noise by precisely controlling a temperature of a sorbent to a target temperature for desorption and preventing thermal degeneration of an adsorbed sample and the sorbent, an analysis device comprising the same, and an analysis method using the same.

Yet still another object of the present invention is to provide a sample concentrator tube having a heat-resistant planar heating element adhered thereto and capable of rapidly performing heating while precisely controlling a temperature and having excellent reproducibility, an analysis device comprising the same, and an analysis method using the same.

Yet still another object of the present invention is to provide a sample concentrator tube having a heat-resistant planar heating element adhered thereto and being inexpensive, economical, and excellent in energy efficiency, an analysis device comprising the same, and an analysis method using the same.

### [Technical Solution]

In one general aspect, a sample concentrator tube according to claim 1 is provided.

The tube may be made of an electrically insulating material or the outer peripheral surface of the tube on which the heating layer is formed may include an electrical insulating layer.

The tube may have a tubular structure including an inner peripheral surface portion and an outer peripheral surface portion, wherein the inner peripheral surface portion may be made of a metal material, and the outer peripheral surface portion may include an electrical insulation layer formed of a metal oxide obtained by anodization of the inner peripheral surface portion.

The tube may further include a heat-resistant gas-permeable sealing member formed in an opening portion of the tube and having a plurality of pores.

The heating layer may be formed in a tubular shape to enclose the outer peripheral surface of the tube.

The electrode layer may be formed in a tubular shape to enclose the outer peripheral surface of the heating layer.

The electrode layer includes first and second electrode layer, and the first and second electrode layers are formed to be spaced apart from each other on both end portions of the heating layer.

The planar heating element is formed of a heating composition including a carbon nanotube or a carbon nanotube-metal complex, and a silicone adhesive. Here, the metal may include any one or two or more selected from silver, platinum, gold, copper, nickel, iron, cobalt, and aluminum.

The heating layer may have sheet resistance of 2 to 15 Ω/sq.

The heating layer may have an average thickness of 20 to 100 µm.

The sample concentrator tube may further include a temperature measurement portion provided on the outer peripheral surface or an inner peripheral surface of the tube.

The sorbent allowing the volatile material to be adsorbed or desorbed may be provided on the inner peripheral surface of the tube.

In another general aspect, a volatile material analysis device includes the sample concentrator tube having a heat-resistant planar heating element adhered thereto as described above.

The volatile material analysis device may include: the sample concentrator tube including a temperature measurement portion provided on the outer peripheral surface or the inner peripheral surface of the tube; a detection portion into which a sample is introduced from the sample concentrator tube; and a control portion receiving a measured value for a temperature in the tube from the temperature measurement portion and comparing the measured value with a preset value to control a voltage applied to the electrode layer.

In still another general aspect, a volatile material analysis method includes: accommodating a sorbent suitable for a sample to be concentrated in the tube (S1) ; sealing an opening portion of the tube with a heat-resistant gas-permeable sealing member in which pores are formed (S2); allowing a gas including the sample to pass through the inside of the tube to adsorb and concentrate the sample in the sorbent (S3); applying a voltage to the electrode layer to thermally desorb the concentrated sample from the sorbent (S4); and introducing the thermally desorbed concentrated sample into a detection portion to analyze the concentrated sample.

### [Advantageous Effects]

With a sample concentrator tube having a heat-resistant planar heating element adhered thereto according to the present invention, an analysis device comprising the same, and an analysis method using the same, a deviation between a measured value and an actual temperature of a sample may be minimized, thereby making it possible to precisely control a temperature to a target temperature.

With the sample concentrator tube according to the present invention, the analysis device comprising the same, and the analysis method using the same, since even though a sorbent is positioned at any position from an inner peripheral surface of a tube to a central portion of the tube, thermal energy may be effectively transferred from an outer surface portion of the tube, a local temperature difference between sample accommodating spaces in the tube may be minimized, thereby making it possible to allow a plurality of sorbents positioned at some sites of the sample accommodating spaces to have a uniform temperature.

The sample concentrator tube according to the present invention, the analysis device comprising the same, and the analysis method using the same may uniformly and precisely control a temperature to a target temperature, such that thermal degradation of the sorbent for adsorbing the sample may be prevented, and chemical noise may be minimized.

The sample concentrator tube according to the present invention, the analysis device comprising the same, and the analysis method using the same may also rapidly raise a temperature to a desorption temperature, and reproducibility may be excellent.

The sample concentrator tube according to the present invention, the analysis device comprising the same, and the analysis method using the same may be inexpensive and economical, and energy efficiency may be excellent.

Even though effects are not explicitly described in the present invention, the effects described in the specification and expected by technical features of the present invention and inherent effects thereof are treated as described in the specification of the present invention.

### [Description of Drawings]

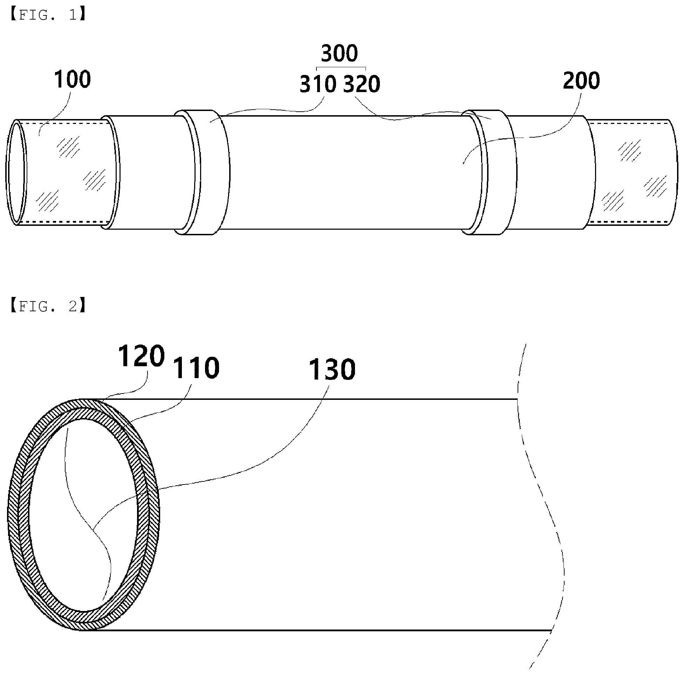

FIG. 1 is a view illustrating a sample concentrator tube according to the present invention.
FIG. 2 is a cross-sectional perspective view illustrating the sample concentrator tube according to the present invention.
FIG. 3 is a cross-sectional perspective view illustrating the sample concentrator tube according to the present invention, including a tube having a large number of sample accommodating spaces formed by partition walls.
FIG. 4 is an actual image of a sample concentrator tube having a heat-resistant planar heating element adhered to a glass tube, according to the present invention.
FIG. 5 is an actual image taken using an infrared camera in a heating state of the sample concentrator tube having a heat-resistant planar heating element adhered thereto, shown in FIG. 4.

### [Detailed Description of Main Elements]

| | | | |
|---|---|---|---|
| 100: | tube | 110: | inner peripheral surface portion |
| 120: | outer peripheral surface portion | 130: | sample accommodating space |
| 131: | first sample accommodating space | 132: | second sample accommodating space |
| 133: | third sample accommodating space | 140: | partition wall |
| 200: | heating layer | 300: | electrode layer, |
| 310: | first electrode layer | 320: | second electrode layer |

### [Best Mode]

Hereinafter, a sample concentrator tube having a heat-resistant planar heating element adhered thereto, an analysis device comprising the same, and an analysis method using the same according to the present invention will be described in detail with reference to the accompanying drawings.

Further, a singular form used in the present specification includes a plural form unless specially described in the text.

Terms themselves indicating respective steps such as s1, s2, s3, ...; a1, a2, a3, ...; b1, b2, b3, ...; a, b, c, ..., and the like, described in the present specifications are used in order to indicate certain steps, units, and the like, but it is not to be interpreted that respective terms refer to order relationships of respective subjects to be indicated by the terms.

Unless specifically mentioned, a unit of % used in the present specification without particular description refers to weight %.

A term such as "layer" or "film" described in the present specification refers to that each material forms a continuum and has a dimension in which thickness is relatively small as compared to width and length. Therefore, it is not to be interpreted that the term such as "layer" or "film" refers to a two-dimensional flat plane.

A sample concentrator tube having a heat-resistant planar heating element adhered thereto according to the present invention includes a tube 100 having an internal structure in which a sorbent adsorbing a volatile material is filled; a heating layer 200 including a heat-resistant planar heating element adhered to an outer peripheral surface of the tube 100; and an electrode layer 300 formed on an outer peripheral surface of the heating layer 200.

The volatile material, which is a target to be analyzed in a general sample analysis device, refers to a material including an inorganic compound, an organic compound, or the like, that may exist in a gas phase. Specifically, examples of the volatile material include organic compounds including hydrocarbon compounds that may be volatilized in the air, and as specific examples, there are infinitely many kinds of organic compounds such as benzene, formaldehyde, xylene, ethylene, styrene, acetaldehyde, and the like. As examples of the inorganic compounds, there are various materials such as hydrochloric acid, hydrofluoric acid, ammonia, hydrogen sulfide, and the like. That is, the volatile material described in the present specification may collectively indicate all detectable materials which have volatility and may exist in a gas phase.

A geometric structure of the tube 100 is not limited as long as the tube 100 has a sample accommodating space 130 therein and has an open portion which a sample may be introduced into the sample accommodating space 130. That is, the tube 100 may literally refer to a tubular structure, and in this case, both ends of the tube may be opened. A shape of one end of the tube 100, that is, a shape of a cross-sectional surface of the tube in a direction vertical to a length direction of the tube 100 is not limited, but may be generally circular shape. The shape may be various shapes such as an oval shape, an n-gonal shape (n is 2 or more), a star shape, a shape in which a plurality of arcs having different degrees of curvature are connected to each other, and a shape in which one or more lines and one or more arcs are connected to each other, in addition to the circular shape.

Although not particularly limited, an inner diameter of the tube 100 is 1 to 5 mm, which is preferable in that thermal energy may be efficiently and effectively transferred to the sample, but the present invention is not necessarily limited thereto. In addition, since a length and a thickness of the tube 100 may be suitably adjusted depending on a scale or specific use (for example, portable use, experimental use, large-scale facility measurement, or the like), the length and the thickness are not particularly limited. As a specific example, the tube 100 may have an outer diameter of 2 to 7 mm.

A material of the tube 100 may be largely divided into a transparent material and an opaque material from an optical point of view. A transparent material may be selected in view of easy identification of a sample or a sorbent, and specific examples thereof may include glass having heat resistance to withstand a temperature of 250° C and preferably 350° C or higher, a heat-resistant transparent polymer material, and the like. Specific examples of the opaque material may include a ceramic material, a metal material, a metal oxide material, and a thermosetting polymer material, and the like.

It is preferable that the tube has an insulation property so that a current applied to an electrode is not transferred to the tube 100 through the heating layer 200. When the material of the tube 100 is a metal, an outer peripheral surface of a metal tube 100 on which the heating layer 200 is formed includes an electrical insulating layer, that is, an insulating layer formed to cover a metal layer. In a case where the current is applied to the tube 100 through the heating layer 200 through the electrode, that is, in a case where the current is applied to the tube having a resistance value close to 0, there is a problem in that a circuit may burn out and thus it is impossible to heat the heating layer. As a non-restrictive example, a member having a structure preventing the current applied to the heating layer 200 from flowing to the tube 100 or member playing the above-mentioned role may be added, and in a case where the problem described above does not occur, the material of the tube 100 itself is not limited. Here, the term "insulation" refers to a property that electricity is not substantially conducted, that is, typically electrical resistance is high.

As described above, it is preferable that the outer peripheral surface of the tube 100 on which the heating layer 200 is formed has an insulation property, and in view of further improving energy efficiency, precision in controlling a temperature, reproducibility, and the like, it is preferable that the tube 100 has a structure including a metal having high thermal conductivity and an electrical insulating layer including a porous anodic oxide film (PAOF) in which the metal is anodized. As a preferable example, the tube 100 may have a tubular structure including an inner peripheral surface portion 110 and an outer peripheral surface portion 120, wherein the inner peripheral surface portion 110 may be an aluminum metal, and the outer peripheral surface portion 120 may be an aluminum-based porous anodic oxide film formed by anodization of the aluminum metal.

Further, in a case where the tube is formed of the aluminum metal, as a preferable example, thicknesses of the inner peripheral surface portion 110 and the outer peripheral surface portion 120 are not limited, but it is preferable that the thickness of the inner peripheral surface portion 110 is 200 to 1000 µm, and the outer peripheral surface portion 120 is 0.1 to 20 µm, specifically 0.3 to 10 µm, and more specifically 0.5 to 5 µm. In this case, it is possible to minimize a flow of the current to the inner peripheral surface portion 110 while improving precision in controlling the temperature, such that energy efficiency may be excellent, and precision in controlling the temperature may be further improved.

The aluminum-based porous anodic oxide film formed by anodization may be formed by an anodization technology, which is one of surface treatment technologies of a metal, and a tube 100 including a metal surface in which various and regular nano-structures are formed using this technology may be used.

As a preferable example, an inner portion of the tube 100, that is, the sample accommodating space 130 may include first to n-th sample accommodating spaces formed by one or two or more partitions 140 walls provided therein. Here, n is a natural number of 2 or more, and as a specific example, n may be selected from 2 to 20, but is not limited thereto. Here, as a material of the partition wall 140, a material equal to or different from that of the tube described above may be used, and a material having excellent thermal conductivity such as a metal, or the like, may be preferably used. In order to detect various materials existing at a trace amount in the air with high sensitivity and lower a limit of detection, there is a need to pass, adsorb, desorb, and analyze a large amount of air in the tube. To this end, a large amount of sorbent may be used, and it is thus required to increase an inner diameter of the tube. However, in this case, since thermal energy transferred between sorbents positioned at respective portions is decreased from the inner peripheral surface portion of the tube to a central portion of the tube, a local temperature difference may be generated. Therefore, simultaneous thermal desorption of volatile material may be difficult due to a time difference in which heat is transferred to the sorbent, such that there is a limitation in significantly increasing an instantaneous concentration for analysis, and precision may be significantly decreased. However, in a case where the first to n-th sample accommodating spaces (here, n is a natural number of 2 or more) formed by one or two or more partition walls 140 provided therein are included in the tube 100 as described above, thermal energy from the heating layer 200 may be rapidly and effectively transferred to the sorbent filled in each of the sample accommodating spaces through the metal structure having high thermal conductivity. Therefore, the volatile material adsorbed in the sorbent may be simultaneously desorbed, such that a trace amount of volatile material may be analyzed. That is, thermal energy converted in the heating layer 200 may be efficiently transferred to the sorbent in each of the sample accommodating space through the tube having high thermal conductivity without thermal resistance due to the tube 100 adhered thereto, such that thermal desorption of the sample may be performed with high energy efficiency, stability of the sorbent may be increased, and reproducibility may be improved.

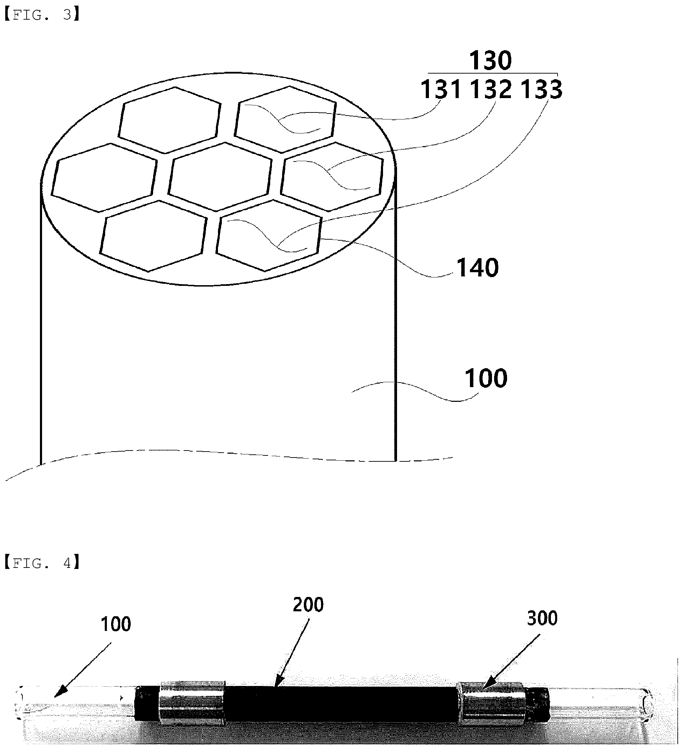

A shape and a structure of the partition wall 140 are not particularly limited as long as the partition wall 140 may be connected to the inner peripheral surface of the tube to partition each of the sample accommodating spaces so that the sorbent is provided in each of the sample accommodating spaces. As a specific example, the partition wall 140 may be formed so that the cross-sectional surface has various shapes such as a circular shape, an oval shape, an n-gonal shape (n is 2 or more), a star shape, a shape in which a plurality of arcs having different degrees of curvature are connected to each other, and a shape in which one or more lines and one or more arcs are connected to each other. As a preferable example, there may be a honeycomb structure formed to include a plurality of hexagonal holes as shown in FIG. 3, and this case is more preferable in view of securing high structural stability in spite of including a plurality of holes in addition the above-mentioned effects.

As described above, the sample concentrator tube according to the present invention includes the sorbent provided in the inner peripheral surface of the tube 100 so that the volatile material is adsorbed or concentrated therein. Generally, after a gas sample is adsorbed in the sorbent in an amount required for detection at a temperature equal to or lower than room temperature, the sample is vaporized in a gas form through thermal desorption by heating to thereby be detected and analyzed. Therefore, it is required to precisely control a temperature of the sample, and in this case, the temperature may be precisely controlled and thermal damage of the sorbent may be prevented by using the sample concentrator tube having a heat-resistant planar heating element adhered thereto according to the present invention. A specific kind of sorbent is not particularly limited as the kind of sorbent is well-known in the field of analysis technology through sample concentration. As a specific example, a sorbent material having a specific surface area of 10 to 2000 m²/g and a density of 0.2 to 0.8 g/cm³ may be used, and examples thereof may include a thermosetting polymer material such as an aromatic polymer or the like and carbon material such as active carbon, graphite, or the like.

As an example of the present invention, the inner peripheral surface of the tube 100 may be subjected to hydrophobic or hydrophilic surface-treatment so that the sample or the sorbent is not adsorbed. Examples of hydrophobic surface-treatment may include coating treatment using a fluorine-based compound, and the like, and examples of hydrophilic surface-treatment may include plasma treatment using steam, nitrogen, or the like, acid treatment, and the like. However, since these treatments are provided only as an example and specific examples of various treatment methods are referred in known documents, the present invention is not limited thereto.

Although the sorbent may be repeatedly used, in a case where the sorbent is frequently used, since thermal damage or degradation may occur as described above, there is a need to periodically change the sorbent. Therefore, the concentrator tube is required to be easily detached from an analysis device and be easily attached thereto, a structure that is not complicated is required so that problems that may occur in this time are significantly decreased, and in particular, strong adhesion of each layer capable of withstanding large physical impact is required. Therefore, the concentrator tube having a heat-resistant planar heating element adhered thereto according to the present invention includes a planar heating layer 200 including a carbon nanotube or a carbon nanotube-metal complex, high stability may be expected in spite of various variables.

The sorbent may be filled and accommodated in the tube 100, and may be filled that the inner portion of the tube 100 is sealed or not sealed.

As described above, since the sample is introduced into the inner portion of the tube 100 and adsorbed in the sorbent, it is preferable that the open portion of the tube 100 may be sealed as needed, and as a means for sealing, the concentrator tube having a heat-resistant planar heating element adhered thereto according to the present invention may further include an openable and closable sealing member. Specifically, the concentrator tube having a heat-resistant planar heating element adhered thereto according to the present invention may further include a heat-resistant gas-permeable sealing member formed in the open portion, specifically, both end portions of the tube 100 and having a plurality of pores formed therein. The sealing member may be bonded to both opened ends of the tube 100 to seal the inner portion of the tube 100, and may be accommodated both end portions of the tube, for example, inwardly from both ends to seal the inner portion of the tube 100. A size of the pore is not limited as long as air may pass through the pore and the sorbent, or the like may be fixed. Further, a material of the sealing member is not limited as long as the sealing member may be used at a temperature equal to or higher than a desorption temperature. For example, a glass fiber, or the like, may be used.

The heating layer 200 includes a planar heating element including one or more selected from a carbon nanotube and a carbon-nanotube-metal complex, and as an example, the heating layer may be formed of a nanotube-based heat-resistant planar heating element. The planar heating element means that the planar heating member may stably generate heat to a temperature of about 350°C when a current is applied thereto, and the planar heating element may be formed on the heating layer to have a layered structure. A formation method thereof is not limited. For example, the planar heating element may be a film (thin layer) formed by applying and drying (thermally-treating) a liquid composition of the planar heating element. Here, since application conditions (temperature, humidity, time, and the like), drying (thermal treatment) conditions (temperature, humidity, time, and the like) are appropriated by the skilled in the art, these conditions are not limited.

Specific examples of the planar heating element described above are disclosed in KR10-1447478B1, KR10-1313149B1, and the like, and for example, the heating layer 200 may include a planar heating element formed of AccuPaste^{™} CNT Heating Paste (TC-1010, Bioneer), or the like. However, this is described only as a preferable example, other various planar heating elements may be used.

The planar heating element is formed of a heating composition to be described below. The heating composition includes a carbon nanotube or a carbon nanotube-metal complex; and a silicone adhesive. Specifically, the heating composition may include 20 to 80 wt% of the carbon nanotube or the carbon nanotube-metal complex and 20 to 80 wt% of the silicone adhesive. The silicone adhesive may contain 0.1 to 10 wt% of a silanol group based on 100 wt% of the adhesive, and a molar ratio of a phenyl group to a methyl group may be 0.3 to 2.5. A metal contained in the carbon nanotube-metal complex is not particularly limited, and examples thereof may include any one or two more selected from silver, platinum, gold, copper, nickel, iron, cobalt, aluminum, and the like. A content of the metal contained in the carbon nanotube-metal complex may be 1 to 80 parts by weight based on 100 parts by weight of the carbon nanotube-metal complex. In addition, the heating composition may further include any one or two or more selected from an organic binder, a dispersant, an organic solvent, and the like. The organic binder may be any one or more selected from ethylcellulose, nitrocellulose, and mixtures thereof, the dispersant may include any one or two or more selected from the group consisting of phosphorus ester salts of an amino-containing oligomer or polymer, monoesters or diesters of phosphoric acid, acidic dicarboxylic acid monoesters, polyurethane-polyamine adducts, and polyalkoxylated monoamines or diamines, and the organic solvent may include any one or two or more selected from acetone, methyl ethyl ketone, methyl alcohol, ethyl alcohol, isopropyl alcohol, butyl alcohol, ethylene glycol, polyethylene glycol, tetrahydrofuran, dimethylformamide, dimethylacetamide, N-methyl-2-pyrrolidone, hexane, cyclohexanone, toluene, chloroform, dichlorobenzene, dimethylbenzene, trimethylbenzene, pyridine, methylnaphthalene, nitromethane, acrylonitrile, octadecylamine, aniline, dimethyl sulfoxide, diethylene glycol ethyl ether, and terpineol. In a case where the heating composition further includes the organic binder, the dispersant, the organic solvent, or the like, a composition ratio thereof is not particularly limited. As an example, the heating composition may include 1 to 50 wt% of the carbon nanotube or the carbon nanotube-metal complex, 1 to 20 wt% of the organic binder, 1 to 30 wt% of the silicone adhesive, 1 to 20 wt% of the dispersant, and 1 to 90 wt% of the organic solvent. The heating layer 200 including the planar heating element may be formed by coating the heating composition as described above on the tube 100, or the heating layer 200 including the planar heating element may be formed by a method such as adhesion of a heating sheet made of the heating composition. However, this case is described as a preferable example, and the present invention is not limited thereto.

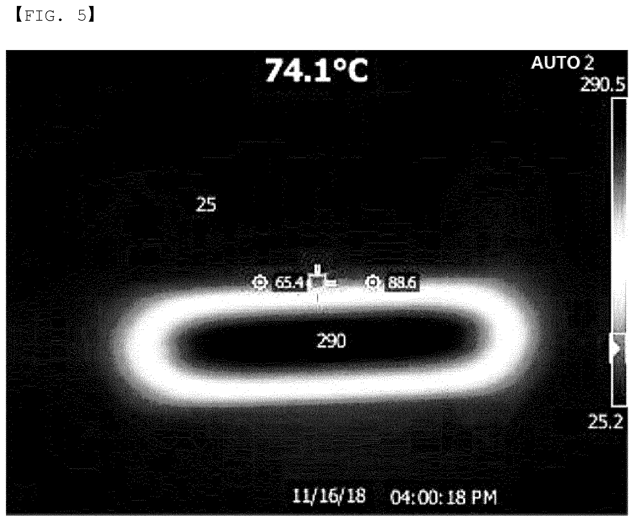

In a sample concentrator tube having a shape in which a heating wire is wound therearound in the related art, the heating wire and a tube 100 are not closely adhered to each other by linear expansion of the heating wire but are partially spaced apart from each other, and heat resistance in a spaced portion therebetween is thus increased, such that there are problems in that it takes a long time to heat the tube and a heating rate becomes non-uniform. In the present invention, the heating layer 200 is uniformly adhered to the tube 100, such that thermal energy may be rapidly and uniformly transferred as shown in FIG. 5. The heating layer 200 includes the heat-resistant planar heating element which is formed of a composition including the carbon nanotube or the carbon nanotube-metal complex and the silicone based adhesive. Further, in a case where the heating layer 200 includes the carbon nanotube-metal complex, the metal may include any one or two or more selected from silver, platinum, gold, copper, nickel, iron, cobalt, aluminum, and the like.

The lower the sheet resistance of the heating layer 200, the better. For example, the heating layer 200 may have sheet resistance of 2 to 15 Ω/sq, but this is only an example and the present invention is not limited thereto.

In an embodiment of the present invention, the heating layer 200 is formed to enclose the outer peripheral surface of the tube 100, and the electrode layer 300 is formed to enclose an outer peripheral surface of the heating layer 200. Here, the heating layer 200 and the electrode layer 300 may have tubular structures in which the outer peripheral surfaces of respective targets may be completely enclosed and may not be completely enclosed. As a preferable example, the heating layer 200 may be formed in a tubular shape to enclose the outer peripheral surface of the tube 100, and the electrode layer 300 may also be formed in a tubular shape to enclose an outer peripheral surface of the heating layer 200.

A thickness of the heating layer 200 is in inversely proportional to electric resistance, and is not limited as long as the heating layer 200 receives electric energy from the electrode layer 300, converts the electric energy into thermal energy, and transfers thermal energy to the tube 100. For example, the thickness of the heating layer 200 may be 20 to 100 µm. However, this is described as a specific example, and the present invention is not limited thereto.

A material of the electrode layer 300 and a structure or a shape in which the electrode layer 300 is formed on the heating layer 200 are not particularly limited as long as the electrode layer 300 may apply a voltage to the heating layer 200, but the electrode layer 300 includes a first electrode layer 310 (300) and a second electrode layer 320 (300). Here, the first electrode layer 310 (300) and the second electrode layer 320 (300) are formed to be spaced apart from each other on both end portions of the heating layer 200. Formation positions of the first electrode layer 310 (300) and the second electrode layer 320 (300) are not particularly limited as long as a voltage may be applied to each electrode layer 300 to heat the heating layer 200. Further, since a spaced distance between the first electrode layer 310 (300) and the second electrode layer 320 (300) may be adjusted in consideration of various variables such as lengths of the tube 100 and the heating layer 200, an area of each electrode layer 300, and the like, the spaced distance is not particularly limited.

Any electrode layer 300 formed of an electrode generally used may be used. For example, the electrode layer 300 may be formed of a conductive material such as copper, iron, or the like, but the present invention is not limited thereto. Further, in some cases, a precious metal layer covering the metal layer may be further formed in view of improving energy efficiency. Examples of a metal used in the precious metal layer may include gold, platinum, and the like, but in addition to these metals, any precious metal may be used without limitation as long as the metal may improve energy efficiency.

A thickness of the electrode layer 300 is not limited as long as the electrode layer 300 may be stably bonded to the tube and may apply electricity to the heating layer. For example, the thickness may be 50 µm to 5 mm, specifically, 100 µm to 1,000 µm, but is not limited thereto.

The electrode layer 300 may be formed to come in contact with the heating layer 200 by various methods. For example, the electrode layer 300 may be formed using a plating method, a brush plating method, a vapor deposition method, and the like, or formed by fixing a metal ring with conductive epoxy, or the like, but may also be formed by various methods in addition to the above-mentioned methods. Therefore, the present invention is not limited thereto.

The sample concentrator tube having a heat-resistant planar heating element adhered thereto according to an embodiment of the present invention may further include an insulating layer for protecting the heating layer on the outer peripheral surface of the heating layer 200.

The sample concentrator tube having a heat-resistant planar heating element adhered thereto according to an embodiment of the present invention may further include a temperature measurement portion provided on the outer peripheral surface or the inner peripheral surface of the tube 100. As the temperature measurement portion, a sensor capable of sensing a temperature may be used. As a specific example, a contact temperature sensor including any one or two or more selected from a thermocouple temperature sensor, a resistive temperature detector (RTD), a thermistor temperature sensor, and the like, or any one or more selected among non-contact temperature sensor including infrared temperature sensors, and the like may be used.

A volatile material analysis device according to the present invention may include the above-mentioned sample concentrator tube having a heat-resistant planar heating element adhered thereto. In detail, the volatile material analysis device according to the present invention may include the sample concentrator tube having a heat-resistant planar heating element adhered thereto, including a temperature measurement portion provided on the outer peripheral surface or the inner peripheral surface of the tube 100; a detection portion into which a sample is introduced from the sample concentrator tube; and a control portion receiving a measured value for a temperature in the tube from the temperature measurement portion and comparing the measured value with a preset value to control a voltage applied to the electrode layer 300.

Since the detection portion may refer to a device capable of detecting and analyzing a sample and is widely known in a gas sample analysis technology field, the detection portion is not limited. As an example, the detection portion may include a detection/analysis device such as a gas chromatography (GC), a mass spectrometer (MS), an ion mobility spectrometer, or the like.

The control portion may monitor a temperature through the temperature measurement portion, and control a temperature of the tube 100 to a target temperature by feedback through a current or voltage control device, or the like. Specifically, when the measured value from the temperature measurement portion is smaller than the preset value, the control portion may serve to increase a voltage applied to the electrode layer 300, and when the measured value from the temperature measurement portion is larger than the preset value, the control portion may serve to decrease a voltage applied to the electrode layer 300.

A volatile material analysis method according to an embodiment of the present invention may include: accommodating a sorbent suitable for a sample to be concentrated in a tube 100 (S1); sealing an opening portion of the tube 100 with a heat-resistant gas-permeable sealing member in which pores are formed (S2); allowing a gas including the sample to pass through the inside of the tube 100 to adsorb and concentrate the sample in the sorbent (S3); applying a voltage to an electrode layer 300 to thermally desorb the concentrated sample from the sorbent (S4); and introducing the thermally desorbed concentrated sample into a detection portion to analyze the concentrated sample (S5).

In S3, a temperature at the time of concentrating the sample is not limited as long as the sample may be concentrated to thereby be adsorbed in the sorbent in a liquid or solid phase, and generally, the temperature may be room temperature, for example, 0 to 25°C. However, the temperature is not particularly limited as long as the sample may be adsorbed.

In S4, a temperature for thermal desorption of the sample is not limited as long as the sample may be desorbed from the sorbent to thereby be introduced into the outside. For example, the temperature may be 100 to 350°C, but since the temperature may be changed depending on the kind of sample, the temperature is not limited thereto.

## Claims

1. A sample concentrator tube comprising:
a tube (100) having an internal structure in which a sorbent adsorbing a volatile material is filled;
a heating layer (200) including a heat-resistant planar heating element adhered to an outer peripheral surface of the tube; and
an electrode layer (300) formed on an outer peripheral surface of the heating layer (200),
wherein the heating layer (200) receives electric energy from the electrode layer (300), converts the electric energy into thermal energy, and transfers the thermal energy to the tube (100),
wherein the electrode layer (300) includes first (310) and second (320) electrode layer, and the first and second electrode layers (310, 320) are formed to be spaced apart from each other on both end portions of the heating layer (200), and
wherein the planar heating element is formed of a heating composition including a carbon nanotube or a carbon nanotube-metal complex; and a silicone adhesive.

2. The sample concentrator tube of claim 1, wherein the tube (100) is made of an electrical insulating material or the outer peripheral surface of the tube (100) on which the heating layer (200) is formed includes an electrical insulating layer.

3. The sample concentrator tube of claim 2, wherein the tube (100) has a tubular structure including an inner peripheral surface portion (110) and an outer peripheral surface portion (120), and
the inner peripheral surface portion (110) is made of a metal material, and the outer peripheral surface portion (120) is an electrical insulating layer formed of a metal oxide obtained by anodization of the inner peripheral surface portion (110).

4. The sample concentrator tube of claim 1, wherein the tube (100) further includes a heat-resistant gas-permeable sealing member in which a plurality of pores for fixing the sorbent are formed.

5. The sample concentrator tube of claim 1, wherein the heating layer (200) is formed in a tubular shape to enclose the outer peripheral surface (120) of the tube (100), and the electrode layer is formed in a tubular shape to enclose the outer peripheral surface (120) of the heating layer (200).

6. The sample concentrator tube of claim 1, wherein a metal of the carbon nanotube-metal complex includes any one or two or more selected from silver, platinum, gold, copper, nickel, iron, cobalt, and aluminum.

7. The sample concentrator tube of claim 6, wherein the heating layer (200) has sheet resistance of 2 to 15 Ω/sq.

8. The sample concentrator tube of claim 1, wherein the heating layer (200) has an average thickness of 20 to 100 µm.

9. The sample concentrator tube of claim 1, further comprising a temperature measurement portion provided on the outer peripheral surface (120) or an inner peripheral surface (110) of the tube.

10. The sample concentrator tube of claim 1, wherein the sorbent allowing the volatile material to be adsorbed or desorbed is provided on the inner peripheral surface (110) of the tube (100).

11. A volatile material analysis device comprising the sample concentrator tube of claims 1 to 10.

12. The volatile material analysis device of claim 11, comprising:
the sample concentrator tube including a temperature measurement portion provided on the outer peripheral surface (120) or the inner peripheral surface (110) of the tube;
a detection portion into which a sample is introduced from the sample concentrator tube; and
a control portion receiving a measured value for a temperature in the tube (100) from the temperature measurement portion and comparing the measured value with a preset value to control a voltage applied to the electrode layer (300).

13. A volatile material analysis method using the sample concentrator tube of claims 1 to 12, comprising:
accommodating a sorbent suitable for a sample to be concentrated in the tube (S1);
sealing an opening portion of the tube with a heat-resistant gas-permeable sealing member in which pores are formed (S2);
allowing a gas including the sample to pass through the inside of the tube to adsorb and concentrate the sample in the sorbent (S3);
applying a voltage to the electrode layer to thermally desorb the concentrated sample from the sorbent (S4); and
introducing the thermally desorbed concentrated sample into a detection portion to analyze the concentrated sample.

## Patentansprüche

1. Probenkonzentratorrohr, umfassend:
ein Rohr (100) mit einer inneren Struktur, worin ein Sorptionsmittel gefüllt ist, das einen flüchtigen Stoff adsorbiert;
eine Heizschicht (200), die ein hitzebeständiges flächiges Heizelement enthält, das an einer äußeren Umfangsfläche des Rohrs haftet; und
eine Elektrodenschicht (300), die auf einer äußeren Umfangsfläche der Heizschicht (200) ausgebildet ist,
wobei die Heizschicht (200) elektrische Energie von der Elektrodenschicht (300) empfängt, die elektrische Energie in thermische Energie umwandelt und die thermische Energie auf das Rohr (100) überträgt,
wobei die Elektrodenschicht (300) eine erste (310) und eine zweite (320) Elektrodenschicht umfasst, und die erste und die zweite Elektrodenschicht (310, 320) so ausgebildet sind, dass sie an beiden Endabschnitten der Heizschicht (200) voneinander beabstandet sind, und
wobei das flächige Heizelement aus einer Heizzusammensetzung gebildet ist, die ein Kohlenstoffnanoröhrchen oder einen Kohlenstoffnanoröhrchen-Metall-Komplex und einen Silikonklebstoff enthält.

2. Probenkonzentratorrohr nach Anspruch 1, wobei das Rohr (100) aus einem elektrisch isolierenden Material hergestellt ist oder die äußere Umfangsfläche des Rohrs (100), auf der die Heizschicht (200) ausgebildet ist, eine elektrisch isolierende Schicht enthält.

3. Probenkonzentratorrohr nach Anspruch 2, wobei das Rohr (100) eine röhrenförmige Struktur mit einem inneren Umfangsoberflächenteil (110) und einem äußeren Umfangsoberflächenteil (120) aufweist, und
der innere Umfangsoberflächenteil (110) aus einem Metallmaterial hergestellt ist und der äußere Umfangsoberflächenteil (120) eine elektrisch isolierende Schicht ist, die aus einem Metalloxid gebildet ist, das durch Anodisierung des inneren Umfangsoberflächenteils (110) erhalten wird.

4. Probenkonzentratorrohr nach Anspruch 1, wobei das Rohr (100) ferner ein hitzebeständiges, gasdurchlässiges Dichtungselement enthält, in dem eine Vielzahl von Poren zur Fixierung des Sorptionsmittels ausgebildet ist.

5. Probenkonzentratorrohr nach Anspruch 1, wobei die Heizschicht (200) rohrförmig ausgebildet ist, um die äußere Umfangsfläche (120) des Rohrs (100) zu umschließen, und die Elektrodenschicht rohrförmig ausgebildet ist, um die äußere Umfangsfläche (120) der Heizschicht (200) zu umschließen.

6. Probenkonzentratorrohr nach Anspruch 1, wobei ein Metall des Kohlenstoffnanoröhrchen-Metall-Komplexes eines oder zwei oder mehr Metalle umfasst, die aus Silber, Platin, Gold, Kupfer, Nickel, Eisen, Kobalt und Aluminium ausgewählt sind.

7. Probenkonzentratorrohr nach Anspruch 6, wobei die Heizschicht (200) einen Flächenwiderstand von 2 bis 15 Ω/sq. aufweist.

8. Probenkonzentratorrohr nach Anspruch 1, wobei die Heizschicht (200) eine durchschnittliche Dicke von 20 bis 100 µm aufweist.

9. Probenkonzentratorrohr nach Anspruch 1, ferner umfassend einen Temperaturmessabschnitt, der an der äußeren Umfangsfläche (120) oder einer inneren Umfangsfläche (110) des Rohrs vorgesehen ist.

10. Probenkonzentratorrohr nach Anspruch 1, wobei das Sorptionsmittel, das die Adsorption oder Desorption des flüchtigen Stoffs ermöglicht, an der inneren Umfangsfläche (110) des Rohrs (100) vorgesehen ist.

11. Vorrichtung zur Analyse flüchtiger Stoffe, die das Probenkonzentratorrohr nach einem der Ansprüche 1 bis 10 umfasst.

12. Vorrichtung zur Analyse flüchtiger Stoffe nach Anspruch 11, umfassend:
das Probenkonzentratorrohr, das einen Temperaturmessabschnitt aufweist, der an der äußeren Umfangsfläche (120) oder der inneren Umfangsfläche (110) des Rohrs vorgesehen ist;
einen Nachweisabschnitt, in den eine Probe aus dem Probenkonzentratorrohr eingeführt wird; und
einen Steuerabschnitt, der einen gemessenen Wert für eine Temperatur in der Röhre (100) von dem Temperaturmessabschnitt empfängt und den gemessenen Wert mit einem voreingestellten Wert vergleicht, um eine an die Elektrodenschicht (300) angelegte Spannung zu steuern.

13. Verfahren zur Analyse flüchtiger Stoffe unter Verwendung des Probenkonzentratorrohrs nach einem der Ansprüche 1 bis 12, umfassend:
Unterbringen eines für eine zu konzentrierende Probe geeigneten Sorptionsmittels in dem Rohr (S1);
Abdichten eines Öffnungsabschnitts des Rohrs mit einem hitzebeständigen, gasdurchlässigen Dichtungselement, in dem Poren ausgebildet sind (S2);
ein Gas, das die Probe enthält, durch das Innere des Rohr strömen zu lassen, um die Probe in dem Sorptionsmittel zu adsorbieren und zu konzentrieren (S3);
Anlegen einer Spannung an die Elektrodenschicht zur thermischen Desorption der konzentrierten Probe aus dem Sorptionsmittel (S4); und
Einbringen der thermisch desorbierten konzentrierten Probe in ein Nachweisteil, um die konzentrierte Probe zu analysieren.

## Revendications

1. Tube concentrateur d'échantillons comprenant :
un tube (100) ayant une structure interne dans laquelle est placé un absorbant adsorbant une matière volatile ;
une couche chauffante (200) comprenant un élément chauffant plan résistant à la chaleur collé à une surface périphérique extérieure du tube ; et
une couche d'électrodes (300) formée sur une surface périphérique extérieure de la couche chauffante (200),
dans lequel la couche chauffante (200) reçoit de l'énergie électrique de la couche d'électrodes (300), convertit l'énergie électrique en énergie thermique et transfère l'énergie thermique au tube (100),
dans lequel la couche d'électrodes (300) comprend une première (310) et une deuxième (320) couche d'électrodes, et les première et deuxième couches d'électrodes (310, 320) sont formées pour être espacées l'une de l'autre sur les deux parties d'extrémité de la couche chauffante (200), et
l'élément chauffant plan est constitué d'une composition chauffante comprenant un nanotube de carbone ou un complexe nanotube de carbone-métal, et d'un adhésif à base de silicone.

2. Tube concentrateur d'échantillons de la revendication 1, dans lequel le tube (100) est fait d'un matériau isolant électrique ou la surface périphérique extérieure du tube (100) sur laquelle la couche chauffante (200) est formée comprend une couche isolante électrique.

3. Tube concentrateur d'échantillons de la revendication 2, dans lequel le tube (100) a une structure tubulaire comprenant une partie de surface périphérique intérieure (110) et une partie de surface périphérique extérieure (120), et
la partie de la surface périphérique intérieure (110) est faite d'un matériau métallique, et la partie de la surface périphérique extérieure (120) est une couche d'isolation électrique formée d'un oxyde métallique obtenu par anodisation de la surface périphérique intérieure (110).

4. Tube concentrateur d'échantillons de la revendication 1, dans lequel le tube (100) comprend en outre un élément d'étanchéité perméable aux gaz et résistant à la chaleur, dans lequel sont formés plusieurs pores pour fixer le sorbant.

5. Tube concentrateur d'échantillons de la revendication 1, dans lequel la couche chauffante (200) est formée dans une forme tubulaire pour entourer la surface périphérique extérieure (120) du tube (100), et la couche d'électrode est formée dans une forme tubulaire pour entourer la surface périphérique extérieure (120) de la couche chauffante (200).

6. Tube concentrateur d'échantillons de la revendication 1, dans lequel un métal du complexe nanotube de carbone-métal comprend un ou deux ou plus choisis parmi l'argent, le platine, l'or, le cuivre, le nickel, le fer, le cobalt et l'aluminium.

7. Tube concentrateur d'échantillons de la revendication 6, dans lequel la couche chauffante (200) a une résistance de feuille de 2 à 15 Ω/sq.

8. Tube concentrateur d'échantillons de la revendication 1, dans lequel la couche chauffante (200) a une épaisseur moyenne de 20 à 100 µm.

9. Tube concentrateur d'échantillons de la revendication 1, comprenant en outre une partie de mesure de la température située sur la surface périphérique extérieure (120) ou sur une surface périphérique intérieure (110) du tube.

10. Tube concentrateur d'échantillons de la revendication 1, dans lequel le sorbant permettant à la matière volatile d'être adsorbée ou désorbée est fourni sur la surface périphérique interne (110) du tube (100).

11. Dispositif d'analyse des matières volatiles comprenant le tube concentrateur d'échantillons des revendications 1 à 10.

12. Dispositif d'analyse des matières volatiles de la revendication 11, comprenant :
le tube concentrateur d'échantillons comprenant une partie de mesure de la température située sur la surface périphérique extérieure (120) ou la surface périphérique intérieure (110) du tube ;
une partie de détection dans laquelle un échantillon est introduit à partir du tube concentrateur d'échantillons ; et
une partie de contrôle recevant une valeur mesurée pour une température dans le tube (100) à partir de la partie de mesure de la température et comparant la valeur mesurée à une valeur prédéfinie pour contrôler une tension appliquée à la couche d'électrode (300).

13. Méthode d'analyse des matières volatiles utilisant le tube concentrateur d'échantillons des revendications 1 à 12, comprenant :
accueillir un sorbant adapté à un échantillon à concentrer dans le tube (S1) ;
sceller une partie de l'ouverture du tube avec un élément de scellement perméable aux gaz et résistant à la chaleur, dans lequel des pores sont formés (S2) ;
permettre à un gaz comprenant l'échantillon de passer à travers l'intérieur du tube pour adsorber et concentrer l'échantillon dans le sorbant (S3) ;
appliquer une tension à la couche d'électrode pour la désorber thermiquement l'échantillon concentré du sorbant (S4) ; et
introduire l'échantillon concentré désorbé thermiquement dans une partie de détection pour analyser l'échantillon concentré.
